# FASCICULE DE BREVET EUROPEEN

(11) **EP 1 457 159 B1**
(45) Date de publication et mention de la délivrance du brevet: **21.07.2010**
(21) Numéro de dépôt: 04356033.3
(22) Date de dépôt: 09.03.2004
(51) Int. Cl.: A61B 17/17

(54) **Canon de perçage pour le positionnement d'un implant glénoidien**
Bohrlehre für die Positionierung eines Gelenkpfannen-Implantates
Drill guide for positioning a glenoid implant

(30) Priorité: 10.03.2003 FR 0302936
(43) Date de publication de la demande: 15.09.2004
(60) Demande divisionnaire: 09179648.2
(73) Titulaire: TORNIER, 38330 Saint-Ismier (FR)
(72) Inventeur: Tornier, Alain, 38330 Sain Ismier (FR); Ekelund, Anders, 16761 Bromma (SE); Comtat, Laurent, 38600 Fontaine (FR)
(74) Mandataire: Myon, Gérard Jean-Pierre

(56) Documents cités:
- US-A- 5 030 219
- US-A- 5 769 856
- US-A- 5 800 551
- US-B1- 6 379 386

## Description

La présente invention concerne un ancillaire de pose d'un implant glénoïdien.

Lors de l'implantation d'une prothèse d'épaule, il est parfois nécessaire de poser à l'extrémité articulaire de l'omoplate d'un patient, un implant glénoïdien qui, d'un côté, s'ancre rigidement dans l'omoplate et qui, de l'autre côté, délimite une surface articulaire de coopération avec la tête d'un implant huméral fixé à l'extrémité correspondante de l'humérus du patient. Pour ancrer fermement l'implant glénoïdien dans l'omoplate, cet implant comporte généralement une quille en saillie du reste de l'implant et destinée à être reçue et immobilisée dans un canal creusé à l'intérieur de l'omoplate à l'aide d'un organe approprié, notamment le forêt d'une perceuse à usage médical. Lors de l'application de cet organe sur l'omoplate, il est courant d'utiliser un ancillaire de guidage qui limite les risques de dérapage de la tige ou de la pointe de cet organe.

Le document US-B1-6,379,386 décrit de tels ancillaires de guidage. En particulier, en regard de ses figures 5 à 9, ce document décrit un ancillaire de guidage pour le foret d'une perceuse, comportant une platine destinée à venir en appui surfacique contre la glène de l'omoplate d'un patient et à l'intérieur de laquelle est formé un trou central de guidage du foret de perçage. Grâce à cet ancillaire, le chirurgien est à même de creuser une amorce d'un canal. Ce document décrit également, en regard de ses figures 15 à 17, un autre ancillaire comportant une platine d'appui sur l'omoplate, à l'intérieur de laquelle sont formés trois trous de guidages successifs d'un foret de perçage. La face de la platine tournée vers l'omoplate est pourvue d'une saillie adaptée pour venir se loger dans l'amorce préalablement creusée. Par applications successives de la perceuse au niveau de chacun des trois trous, le chirurgien dégage un volume de matière osseuse important et creuse ainsi un canal de réception suffisamment grand pour la quille de l'implant glénoïdien envisagé dans ce document.

Selon la morphologie du patient, l'état de l'omoplate sur laquelle un implant glénoïdien est destiné à être posé, les conditions opératoires et/ou la forme exacte de l'implant à poser, la direction longitudinale du canal de réception de la quille de l'implant est différente. Le chirurgien a généralement intérêt à creuser un canal dans la direction où l'omoplate du patient est la plus résistante, notamment dans une zone épaisse où l'os est abondant et sain.

Actuellement, la direction pour creuser ce canal est repérée sur l'omoplate soit de façon empirique par le chirurgien, soit, lorsque ce dernier utilise un ancillaire tel que celui décrit dans le document US-6,379,386 mentionné ci-dessus, arbitrairement imposée par la platine de l'ancillaire utilisé. Si le chirurgien tente d'appliquer l'organe de perçage suivant une direction choisie sans utiliser d'ancillaire de guidage, il court le risque de voir l'organe de perçage déraper et/ou creuser un canal dans une direction différente de celle qu'il visait, moins favorable pour la pose et la tenue ultérieure de l'implant glénoïdien.

Le but de la présente invention est de proposer un ancillaire de structure simple et qui permette d'imposer à l'organe de perçage, de façonnage ou analogue utilisé lors de la pose d'un implant glénoïdien, par exemple au foret d'une perceuse à usage médical, une direction d'application choisie par le chirurgien, le cas échéant en préopératoire, sur toute une gamme de directions envisageables, voire pour n'importe quelle direction anatomiquement envisageable.

A cet effet, l'invention a pour objet un ancillaire de pose d'un implant glénoïdien, tel que défini à la revendication 1.

Par sollicitation de ces moyens d'ajustement, le chirurgien est à même de disposer d'un guidage ajusté suivant la direction qu'il aura préalablement choisie, par exemple suite à l'analyse de coupes scanner de l'omoplate réalisées avant l'intervention chirurgicale. Ces moyens d'ajustement étant adaptés pour être reliés de façon amovible à la platine, le chirurgien peut juste avant l'opération, ou si nécessaire en per-opératoire, ajuster la direction de guidage imposée à l'organe de perçage utilisé.

D'autres caractéristiques de cet ancillaire, prises isolément ou selon toutes les combinaisons techniquement possibles, sont spécifiées aux revendications dépendantes 2 à 9.

On propose également ici une méthode de pose d'un implant glénoïdien, dans laquelle :
- on dispose d'au moins une coupe scanner ou analogue de l'omoplate d'un patient,
- on détermine une direction préférentielle d'application sur l'omoplate d'un organe de perçage, de façonnage ou analogue,
- on utilise un ancillaire de guidage qui comporte une platine formant un guide directionnel pour ledit organe de perçage et des moyens d'ajustement de la direction du guide par rapport à la platine, lesquels moyens d'ajustement comportent un élément mobile par rapport à la platine et un élément d'immobilisation de l'élément mobile par rapport à la platine, ledit élément mobile étant pourvu d'un perçage traversant rectiligne, et dans laquelle, avant d'avoir plaqué la platine sur la glène de l'omoplate, on positionne l'élément mobile par rapport à la platine de façon à amener le perçage de l'élément mobile sensiblement suivant la direction préférentielle puis on immobilise l'élément mobile par rapport à la platine par l'intermédiaire de l'élément d'immobilisation,
- on plaque une surface de la platine sur la glène de l'omoplate,
- on introduit l'organe de perçage dans le guide dont la direction est ajustée suivant la direction préférentielle, et
- on actionne l'organe de perçage de façon à agir sur l'omoplate suivant la direction préférentielle.

L'invention sera mieux comprise à la lecture de la description qui va suivre, donnée uniquement à titre d'exemple et faite en se référant aux dessins sur lesquels :
- la figure 1 est une vue en perspective d'un ancillaire non conforme à l'invention ;
- la figure 2 est une vue en élévation, prise selon la flèche II indiquée sur la figure 1, de l'ancillaire appliqué sur une omoplate d'un être humain ;
- la figure 3 est une vue en perspective en éclaté de l'ancillaire de la figure 1 ;
- les figures 4 et 5 sont des coupes selon les plans indiqués IV et V sur la figure 3 ;
- la figure 6 est une vue analogue à la figure 3 d'une variante de l'ancillaire de la figure 1 ;
- la figure 7 est une vue en élévation prise selon la flèche VII indiquée sur la figure 6 ;
- la figure 8 est une vue en perspective d'un ancillaire selon l'invention ;
- la figure 9 est une vue en coupe longitudinale de certains éléments de l'ancillaire de la figure 8, représentés à plus grande échelle ;
- la figure 10 est une vue en élévation, prise selon la flèche X indiquée sur la figure 9, de l'éléments sur lequel pointe cette flèche X ;
- la figure 11 est une vue en élévation, prise selon la flèche XI indiquée sur la figure 9, de l'élément sur lequel pointe cette flèche XI ;
- la figure 12 est une vue en élévation d'un outil utilisé lors de la manipulation de l'ancillaire de la figure 8 ;
- la figure 13 est une vue en élévation de côté de l'ancillaire de la figure 8 ; et
- la figure 14 est une vue de dessus de l'ancillaire de la figure 8.

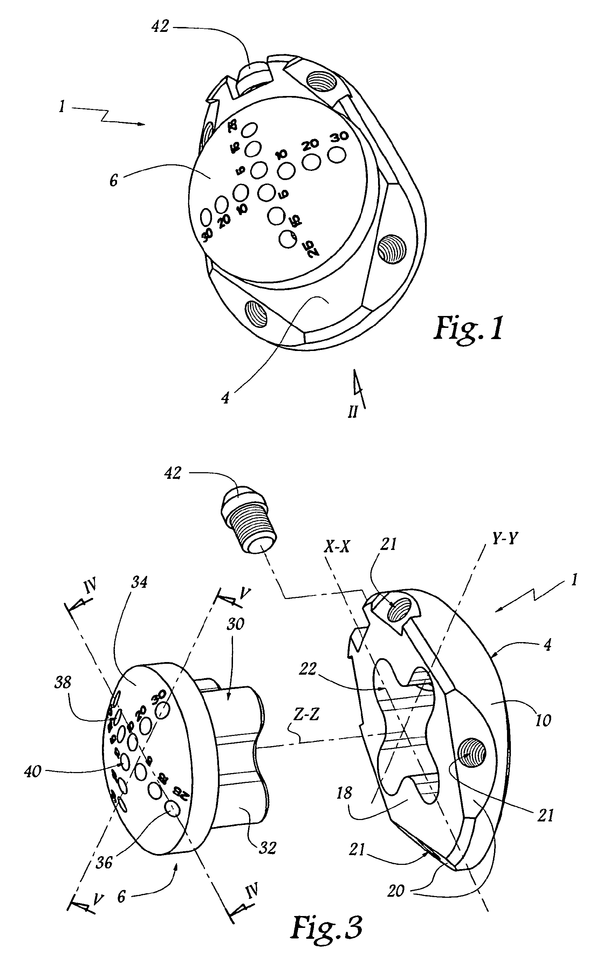

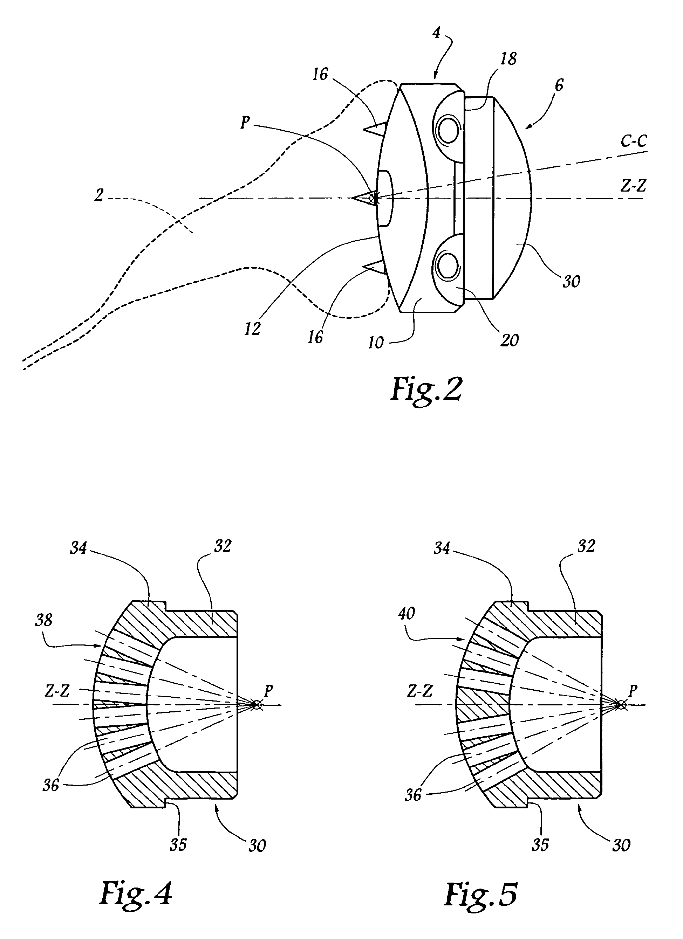

Sur la figure 1 est représenté un ancillaire 1 de pose d'un implant glénoïdien, adapté pour guider directionnellement le foret d'une perceuse à usage médical non représentée. Lors de la pose d'un implant glénoïdien sur l'omoplate d'un patient, en vue de former une prothèse d'épaule, le chirurgien doit creuser dans la glène de l'omoplate un canal de réception pour une quille de l'implant glénoïdien. En fonction de la morphologie du patient, de la géométrie de cette quille, de la pathologie ayant motivée la pose de l'implant, etc., la direction suivant laquelle ce canal est à former est différente. Pour déterminer la direction optimale de ce canal, le terme « optimal » signifiant ici « préférentiel » en considérant l'ensemble des paramètres médicaux portés à la connaissance du chirurgien, ce dernier dispose par exemple d'une série de coupes scanner de l'omoplate sur laquelle il est prévu de poser l'implant et détermine cette direction privilégiée. La localisation exacte du point d'application de l'organe de perçage est secondaire par rapport à la direction privilégiée, étant entendu que ce point d'application se situe dans une zone centrale de la cavité glénoïdienne. La figure 2 représente en pointillé une omoplate 2 d'un patient telle qu'elle apparaît au chirurgien par une coupe scanner. L'axe C-C indique la direction privilégiée choisie par le chirurgien comme étant la direction suivant laquelle l'organe de perçage devrait être appliqué.

A cet effet, l'ancillaire 1 comporte essentiellement une platine 4 sur laquelle sont rapportés des moyens 6 d'ajustement de la direction d'un guide de perçage par rapport à la platine.

Plus précisément, comme représenté sur les figures 1 à 5, la platine 4 est formée d'un corps métallique 10 présentant, suivant une direction notée Z-Z une dimension plus petite que sur les deux directions orthogonales restantes, indiquées par des axes X-X et Y-Y. Par la suite, les termes inférieur et supérieur seront orientés par rapport à l'axe X-X de sorte que, par exemple en regard de la figure 3, le terme supérieur correspond à la partie haute de cette figure, tandis que le terme inférieur correspond à la partie basse.

Le corps 10 de la platine 4 délimite une surface convexe 12 destinée à venir en appui surfacique contre l'omoplate 2, comme représenté sur la figure 2. Avantageusement, la surface 12 est pourvue de picots saillants 16 destinés à faciliter la retenue de la platine par rapport à l'omoplate comme il sera expliqué plus loin.

Le corps 10 délimite une surface 18 opposée à la surface 12, sensiblement plane et à partir de laquelle sont ménagées, aux quatre coins du corps 10, des surfaces biseautées 20. Dans ces surfaces 20 sont formés des évidements taraudés 21, adaptés pour recevoir des moyens (non représentés) de fixation amovible pour un manche de manipulation de la platine connu en soi.

Le corps 10 délimite intérieurement une ouverture traversante 22, d'axe central Z-Z et de profil cruciforme en coupe transversale. Plus précisément, l'ouverture 22 comporte deux paires de lobes diamétralement opposés, les lobes d'une même paire étant alignés respectivement suivant les directions X-X et Y-Y.

Les moyens d'ajustement 6 sont formés par un canon 30 représenté plus en détail sur les figures 3 à 5. Ce canon comporte un corps tubulaire 32 d'axe Z-Z et de profil extérieur sensiblement complémentaire du profil de l'ouverture traversante 22 du corps 10. Le corps 32 est fermé à une de ses extrémités par un fond globalement hémisphérique 34 qui forme, avec la surface extérieure du corps 32, un épaulement plan 35 radialement saillant par rapport à l'axe Z-Z.

A l'intérieur de ce fond 34 sont formés des trous traversants rectilignes 36 dont les axes respectifs sont sensiblement concourants en un point noté P appartenant à l'axe Z-Z. Ces trous 36 se répartissent en une première série 38 de trous qui s'étendent dans un premier plan contenant les axes X-X et Z-Z et correspondant sensiblement au plan de coupe de la figure 4, et une seconde série 40 de trous qui s'étendent dans un second plan contenant les axes Y-Y et Z-Z et correspondant au plan de coupe de la figure 5.

Dans chaque série 38, 40 de trous 36, l'écartement angulaire entre deux trous adjacents situés d'un même côté de l'axe Z-Z vaut sensiblement 10°. L'écartement angulaire entre l'axe Z-Z et l'un des deux trous 36 de la série 38 situés le plus au centre du couvercle 34 vaut environ 5°, tandis que le même écart angulaire pour les deux trous les plus au centre de la série 40 vaut environ 10°

Par coopération du corps tubulaire 32 avec l'ouverture traversante 22 de la platine 10, le canon 30 est à même d'être reçu et immobilisé par rapport à la platine 4, l'épaulement 35 se positionnant alors en butée contre la surface sensiblement plane 18 de la platine, comme représenté sur la figure 2. Le point P est alors inscrit sensiblement dans le prolongement géométrique de la face 12 destinée à venir en appui contre l'omoplate 2.

Dans la partie supérieure de la platine 4, une vis 42 est logée dans un perçage complémentaire s'étendant suivant la direction X-X et débouchant d'un côté sur le chant du corps 10 et de l'autre côté dans l'ouverture 22.

Cette vis permet de retenir suivant l'axe Z-Z le canon 30 dans l'ouverture 22.

L'utilisation de l'ancillaire 1 lors de la pose d'un implant glénoïdien pour prothèse d'épaule est la suivante :

Lorsque le chirurgien a déterminé, par exemple au moyen de coupes scanner de l'épaule d'un patient, la direction privilégiée C-C suivant laquelle il souhaite ancrer l'implant glénoïdien dans l'omoplate et donc appliquer un foret de perçage, il met en appui l'ancillaire 1 contre l'omoplate. Plus précisément, la surface 12 de la platine 4 est plaquée contre la glène osseuse de l'omoplate 2, comme représentée sur la figure 2. A cet effet, l'ancillaire 1 est avantageusement manipulé par un manche relié à la platine 10 au niveau des surfaces biseautées 20. Les picots 16 facilitent l'amarrage de la platine par rapport à l'omoplate.

Alors que le canon 30 est reçu et immobilisé dans l'ouverture 22 de la platine, le chirurgien sélectionne, parmi l'ensemble des trous 36 du canon, l'unique trou dont l'axe longitudinal est le plus proche de la direction préférentielle C-C qu'il a choisie. Si nécessaire, le chirurgien dégage le canon 30 de la platine, le fait pivoter d'un quart de tour puis le remet en place dans l'ouverture cruciforme 22, ce qui permet de disposer de directions de perçage ajustables avec un pas de 5° dans les deux plans contenant respectivement les axes X-X et Z-Z et les axes Y-Y et Z-Z.

Sur la figure 2, la direction choisie C-C forme, dans un plan contenant les axes Y-Y et Z-Z, un écart angulaire d'environ 15° par rapport à l'axe central de la cavité glénoïdienne, de sorte que le chirurgien va utiliser un seul des trous de la série 38 du canon.

Il introduit ensuite dans ce trou le foret d'une perceuse, afin de réaliser un perçage ou au moins une amorce de perçage dans la direction imposée par le trou. Si nécessaire, après avoir dégagé l'ancillaire 1, le chirurgien peut élargir l'orifice percé dans l'omoplate par un foret de plus grand diamètre ou un autre organe de façonnage. Il peut également utiliser une broche de fraisage qui, calée dans l'orifice percé, permet de ménager un plan de résection glénoïdien orthogonal à la direction C-C.

L'ancillaire 1 est ainsi d'utilisation facile et met rapidement à disposition du chirurgien un guide d'application d'un organe, notamment un guide de perçage, dont la direction est ajustée suivant la direction préférentielle que le chirurgien a choisie.

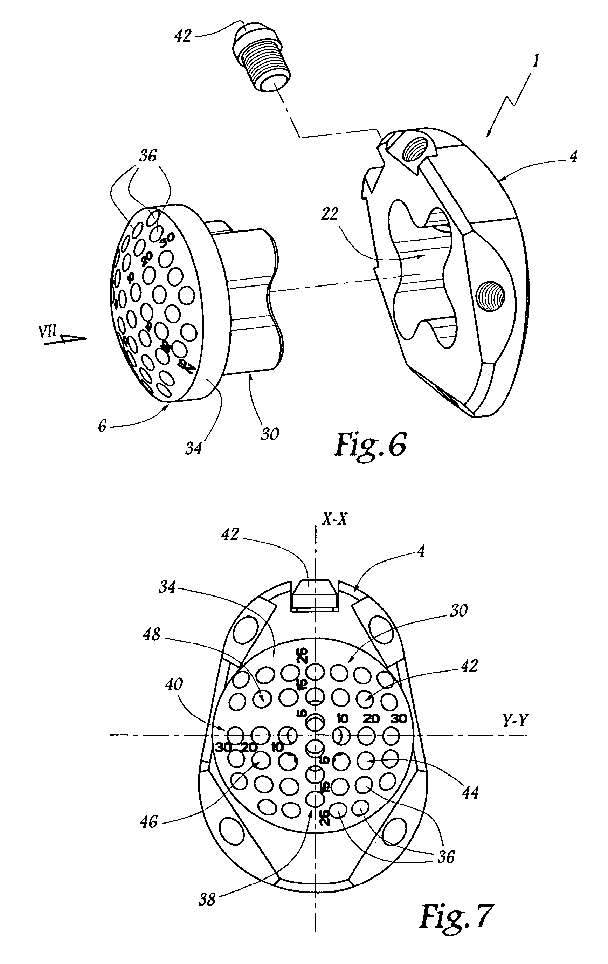

Sur les figures 6 et 7 est représentée une variante de l'ancillaire 1 qui se distingue de l'ancillaire des figures précédentes par le nombre et la disposition des trous de guidage 36 ménagés par le canon 30. En plus des séries de trous 38 et 40, des séries de trous 42, 44, 46 et 48 sont situés respectivement dans les quatre quadrants délimités par les séries de trous 38 et 40, en formant un quadrillage du fond 34 du canon. Cette variante met à la disposition du chirurgien plus de directions de guidage que l'ancillaire des figures 1 à 5.

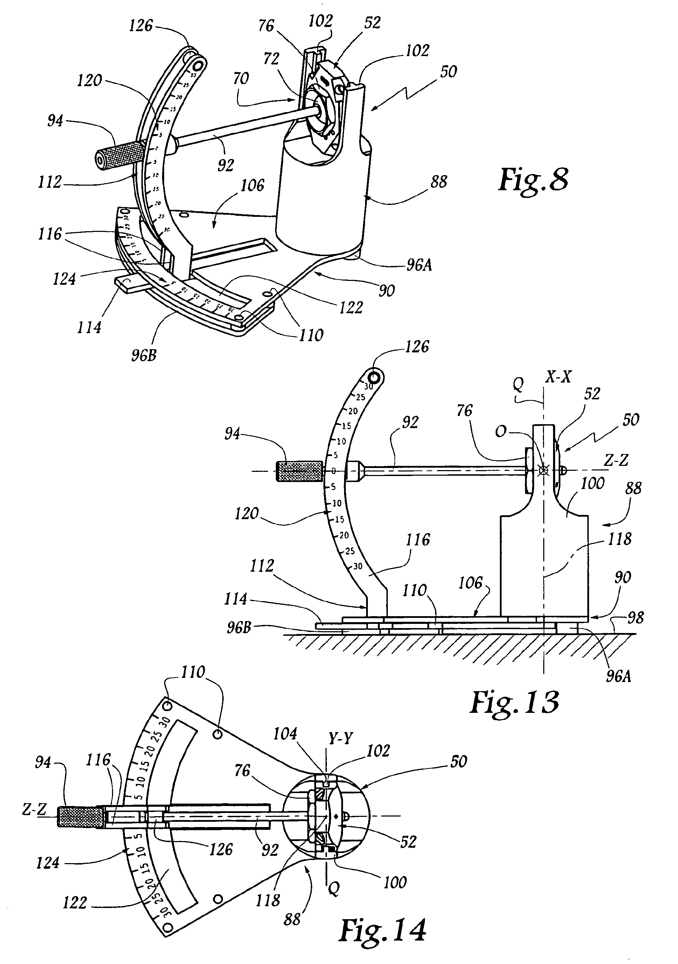

Sur les figures 8 à 14 est représenté un second ancillaire 50 conforme à l'invention. Cet ancillaire est destiné à être utilisé dans le même cadre que l'ancillaire 1 des figures précédentes, c'est-à-dire pour guider la tige d'un organe, notamment le foret d'une perceuse à usage médical, lors de la pose d'un implant glénoïdien d'une prothèse d'épaule.

Comme représenté en détail sur les figures 8 à 11, cet ancillaire 50 comporte une platine 52 formée d'un corps métallique 54 de sensiblement même forme que le corps 10 de la platine 4 et orienté par rapport à des axes X-X, Y-Y et Z-Z définis de la même façon que sur la figure 3.

Le corps 54 délimite une surface convexe 56 destinée à venir s'appuyer contre la glène osseuse d'un patient, avantageusement pourvue de picots d'amarrage 58 sensiblement analogues aux picots 16 de la platine 10, ainsi qu'une surface opposée plane 59. Le corps 54 est également adapté pour être fixé de manière amovible à un manche de manipulation non représenté.

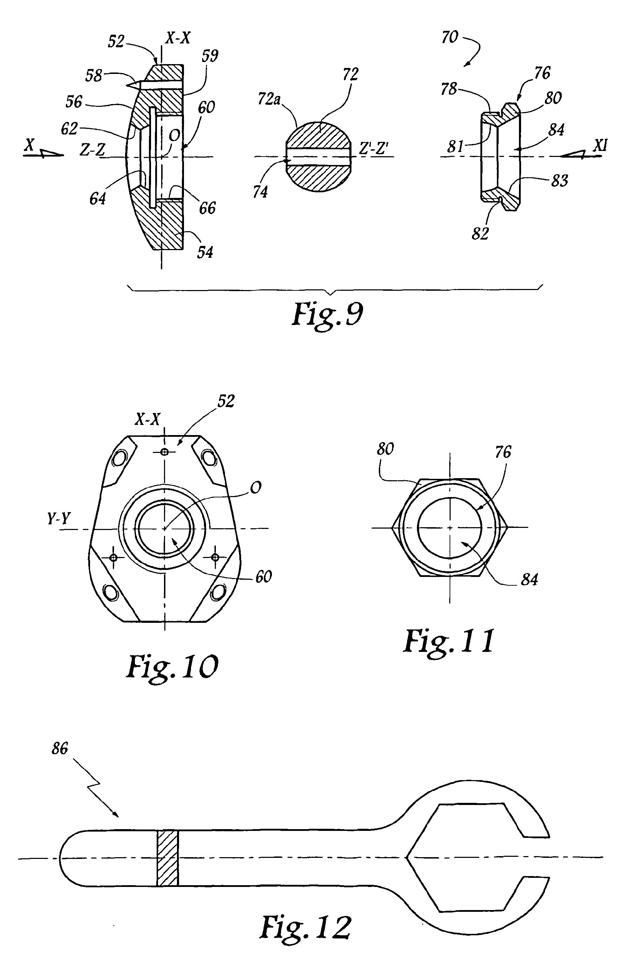

Dans le corps 54 de la platine 52 est formée une ouverture traversante 60 d'axe de révolution Z-Z. Comme représenté sur la figure 9, cette ouverture comporte, successivement depuis la face 56 du corps 54, une partie tronconique 62 qui diverge vers la face 56, une partie 64 en tronc de sphère de centre O et une partie cylindrique 66 taraudée.

L'ancillaire 50 comporte également des moyens 70 d'ajustement de la direction d'un guide de perçage par rapport à la platine 52, comprenant d'une part, une sphère 72 percée de part en part suivant un de ses diamètres en formant un unique perçage cylindrique 74 d'axe Z'-Z', et d'autre part, un écrou 76.

Plus précisément, la sphère 72 présente une surface extérieure 72a sensiblement complémentaire de la partie hémisphérique 64 de l'ouverture 60. L'écrou 76 est formé d'un corps tubulaire 78, d'axe Z-Z et fileté de façon sensiblement complémentaire de la partie cylindrique taraudée 66 de l'ouverture 60, et d'une tête hexagonale 80 qui forme avec le corps tubulaire 78 un épaulement 82 radialement saillant par rapport à l'axe du corps 78. L'écrou 76 ménage intérieurement un alésage 84 formé, du côté du corps tubulaire 78, d'une partie 81 sensiblement en forme de tronc de sphère complémentaire de la surface extérieure de la sphère 72, et, du côté de la tête 80, d'une partie évasée 83.

Par coopération de la surface extérieure 72a de la sphère 72 avec la surface 64 de l'ouverture 60, la sphère 72 est mobile par rapport à la platine 52 à la façon d'une rotule de centre O. De plus, par engagement et coopération des filetages du corps 78 de l'écrou 76 et de la partie taraudée 66 de l'ouverture 60, l'écrou est à même d'immobiliser la sphère 72 par rapport à la platine. La face plane 59 de la platine forme une surface d'arrêt pour l'épaulement 82, évitant ainsi un serrage excessif, voire la détérioration de la sphère par l'écrou serré.

Pour permettre d'appliquer un couple de serrage important sur l'écrou 76, l'ancillaire 50 comporte avantageusement une clef 86, représentée sur la figure 12, pourvue d'une tête évidée d'entraînement de la tête hexagonale de l'écrou.

La sphère 72 étant mobile par rapport à la platine 52 lorsque l'écrou 76 n'est pas serré, cette sphère est adaptée pour être déplacée de façon à ce que l'axe Z'-Z' de son perçage 74 soit ajustée suivant la direction privilégiée C-C choisie préalablement par le chirurgien.

L'ancillaire 50 comporte en outre des moyens 88 de déplacement et de positionnement de la sphère 72 par rapport à la platine 52, représentés sur les figures 8, 13 et 14. Ces moyens comprennent un support rigide 90 d'une part et une tige 92 d'entraînement de la sphère 72 d'autre part, reliée au support 88.

Plus précisément, la tige 92 est adaptée, à une de ses extrémités, pour être enfilée à l'intérieur du perçage 74 de la sphère 72, et comporte à son autre extrémité une poignée moletée 94 de manipulation de la tige, rapportée de façon amovible sur cette tige à la façon d'un écrou.

Le support rigide 90 comporte quant à lui :
- une base d'appui sur une surface sensiblement plane, référencée 98 uniquement sur la figure 13, formée par exemple par une table ou tout plan de travail analogue ; cette base est constituée par exemple d'un pied 96A et d'un talon 96B ;
- une embase 100 de réception et d'immobilisation de la platine 52, pourvue d'un U dont les branches parallèles portent chacune une nervure longitudinale 102 adaptée pour être reçue dans une rainure correspondante 104 formée dans les chants latéraux du corps 54 de la platine, comme représenté sur la figure 14, de façon à positionner le plan Q de la platine, contenant le point O et les axes X-X et Y-Y, sensiblement perpendiculairement à la surface plane 98 ;
- un rapporteur 106 dit horizontal, sous forme d'une plaque sensiblement parallèle à la surface plane 98 ; ce rapporteur horizontal 106 est d'une part solidarisé à l'embase 100 et d'autre part relié fixement au talon 96B de la base d'appui par des pions de fixation 110 ;
- un rapporteur 112 dit vertical, constitué d'une barre plate 114 interposée entre le talon 96B et la plaque du rapporteur horizontal 106, et d'une paire de bras 116 parallèles l'un à l'autre, qui s'étendent transversalement depuis la barre 114 et entre lesquels est retenue la tige 92.

La barre 114 est montée pivotante par rapport au reste du support 90, autour d'un axe de pivotement 118 perpendiculaire à la surface 98 et formé par exemple par un pion cylindrique guidé en rotation dans un évidement correspondant formé dans la plaque du rapporteur horizontal 106 et l'embase 100.

Chaque bras 116 présente suivant sa longueur un profil essentiellement semi-circulaire de centre O et porte des graduations d'angle correspondantes, référencées 120 sur les figures 8 et 13. La graduation zéro correspond à la position de la tige 92 perpendiculaire an plan Q. Au niveau de leurs extrémités reliées à la plaque pivotante 114, les bras 116 sont reçus dans une rainure 122 du rapporteur horizontal 106, semi-circulaire centrée sur l'axe de pivotement 118. La périphérie de la rainure 122 porte des graduations angulaires référencées 124. La graduation zéro correspond à la position de la tige 92 perpendiculaire au plan Q. A leurs extrémités opposées, les bras 116 sont reliés par un pontet de jonction 126.

L'utilisation de l'ancillaire 50 lors de la pose d'un implant glénoïdien pour une prothèse d'épaule est la suivante :

De la même façon que pour l'ancillaire 1 des figures 1 à 5, le chirurgien détermine la direction privilégiée C-C selon laquelle il souhaite procéder au creusement du canal de réception d'une quille de l'implant glénoïdien.

En partant de la configuration de l'ancillaire 50 représentée sur les figures 8, 13 et 14, dans laquelle la platine 52 est immobilisé dans l'embase 100 du support 90, le chirurgien, après avoir si nécessaire desserré légèrement l'écrou 76, entraîne la sphère 72 au moyen de la tige 92. Par déplacement de la poignée moletée 94 vers le haut ou vers le bas par rapport à la surface plane 98, l'inclinaison de la tige dans un plan perpendiculaire à l'axe Y-Y est réglée. Par déplacement de la poignée 94 vers la gauche ou vers la droite dans un plan parallèle à la surface plane 98, l'ensemble du rapporteur vertical 112 pivote autour de l'axe 118 et l'orientation angulaire de la tige dans un plan perpendiculaire à l'axe X-X de la platine est réglée. Le chirurgien s'assure du bon positionnement angulaire de la tige par lecture directe sur les références angulaires correspondantes 120 et 124.

Une fois la tige 92 dirigée suivant la direction choisie, le chirurgien utilise la clef 86 pour serrer fermement l'écrou 76 et ainsi immobiliser la sphère 72 par rapport à la platine 52. La tête de la clef 86 est à cet effet pourvue d'une fente suffisamment large pour permettre de mettre en prise la clef sur la tête 80 de l'écrou sans être gêné par la tige 92.

Il dégage ensuite la platine 52 de l'embase 100, en retirant par exemple à la fois la platine, la sphère 72, l'écrou serré 76 et la tige 92 en dévissant préalablement la poignée 94. Puis la tige 92 est retirée sans difficulté du trou 74.

Ensuite, de la même façon que pour l'ancillaire 1 des figures 1 à 5, le chirurgien place la platine 52 en appui surfacique contre la glène osseuse du patient et introduit à l'intérieur du trou 74 un foret de perçage.

Le second mode de réalisation de l'ancillaire 50 présente l'avantage, par rapport à l'ancillaire 1 des figures 1 à 5, de permettre au chirurgien d'ajuster continûment la direction du guide de perçage suivant toutes les directions anatomiquement concevables, et pas seulement suivant une série discrète de ces directions.

Divers aménagements et variantes aux deux modes de réalisation de l'ancillaire décrits ci-dessus sont en outre envisageables. A titre d'exemple, la face d'appui 12 ou 56 est plane. De plus, les positions des points P et O précités peuvent s'écarter quelque peu de celles décrites plus haut. A titre de variante, les moyens d'ajustement 70 à sphère sont remplacés par un système à glissière permettant l'ajustement directionnel dans un plan unique.

De même, l'ancillaire selon l'invention est à même de guider directionnellement tout organe de perçage, de façonnage ou analogue autre qu'un forêt de perçage, par exemple une broche à impacter ou un clou de marquage.

## Revendications

1. Ancillaire de pose d'un implant glénoïdien, du type comportant une platine (52) qui délimite une surface d'appui (56) destinée à venir s'appuyer contre la glène d'une omoplate (2) d'un patient et qui forme un guide directionnel pour un organe de perçage, de façonnage ou analogue, **caractérisé en ce qu'**il comporte des moyens (70) d'ajustement de la direction du guide par rapport à la platine (52), qui comportent, d'une part, un élément (72) mobile par rapport à la platine et, d'autre part, un élément (76) d'immobilisation de l'élément mobile (72) par rapport à la platine, l'élément mobile étant pourvu d'un perçage traversant (74) rectiligne adapté pour former le guide directionnel pour l'organe de perçage, de façonnage ou analogue.

2. Ancillaire suivant la revendication 1, **caractérisé en ce que** les moyens (70) d'ajustement de la direction du guide sont adaptés pour être reçus et immobilisés dans une ouverture traversante (60) formée dans la platine (52) et débouchant sur la surface d'appui (56) de la platine.

3. Ancillaire suivant l'une des revendications 1 ou 2, **caractérisé en ce que** les moyens d'ajustement (70) permettent d'ajuster la direction du guide par rapport à la platine (52) au moins dans deux plans (X-X, Z-Z), (Y-Y, Z-Z) sensiblement perpendiculaires l'un à l'autre.

4. Ancillaire suivant l'une quelconque des revendications précédentes, **caractérisé en ce que** la direction (Z'Z') du perçage (74) de l'élément mobile (72) est continûment ajustable.

5. Ancillaire suivant l'une quelconque des revendications précédentes, **caractérisé en ce que** l'élément mobile (72) présente une surface (72a) adaptée pour coopérer avec une surface associée (64) de la platine (52).

6. Ancillaire suivant la revendication 5, **caractérisé en ce que** ladite surface (72a) de l'élément mobile (72) adaptée pour coopérer avec la surface associée (64) de la platine (52) est sensiblement sphérique.

7. Ancillaire suivant l'une quelconque des revendications précédentes, **caractérisé en ce qu'**il comporte en outre un outil (86) de commande de l'élément d'immobilisation (76).

8. Ancillaire suivant l'une quelconque des revendications précédentes, **caractérisé en ce qu'**il comprend en outre des moyens (88) de déplacement et de positionnement de l'élément mobile (72) par rapport à la platine (52), adaptés pour amener le perçage (74) de l'élément mobile sensiblement suivant une direction choisie (C-C).

9. Ancillaire suivant la revendication 8, **caractérisé en ce que** les moyens (88) de déplacement et de positionnement de l'élément mobile (72) comportent un support rigide (90) de retenue pour la platine (52) et une tige (92) d'entraînement de l'élément mobile, déplaçable par rapport au support rigide, ledit support étant pourvu de deux rapporteurs (106, 112) adaptés pour rapporter la position angulaire de la tige (92) dans deux plans sensiblement perpendiculaires l'un à l'autre.

## Claims

1. Ancillary tool for positioning a glenoid implant, of the type comprising a plate (52) which defines a bearing surface (56) intended to abut against the glenoid cavity of a patient's scapula and which forms a directional guide for a drilling, fashioning or like member,
**Characterised in that** it comprises means (70) for adjusting the direction of the guide with respect to the plate (52), which comprise, on the one hand, an element (72) mobile with respect to the plate and, on the other hand, an element (76) for immobilizing the mobile element (72) with respect to the plate, the mobile element being provided with a rectilinear through bore (74) adapted to form the directional guide for the drilling, fashioning or like member.

2. The tool of Claim 1, **characterised in that** the means (70) for adjusting the direction of the guide are adapted to be received and immobilized in a through opening (60) formed in the plate (52) and opening out on the bearing surface (56) of the plate.

3. The tool of Claim 1 or 2, **characterised in that** the adjusting means (70) make it possible to adjust the direction of the guide with respect to the plate (52) at least in two planes ((X-X, Z-Z), (Y-Y, Z-Z)) which are substantially perpendicular to each other.

4. The tool of any one of preceding claims, **characterised in that** the direction (Z'-Z') of the bore (74) of the mobile element (72) is continuously adjustable.

5. The tool of any one of preceding claims, **characterised in that** the mobile element (72) presents a surface (72a) adapted to cooperate with an associated surface (64) of the plate (52).

6. The tool of Claim 5, **characterised in that** said surface (72a) of the mobile element (72) adapted to cooperate with the associated surface (64) of the plate (52) is substantially spherical.

7. The tool of any one of preceding claims, **characterised in that** it further comprises a tool (86) for controlling the immobilizing element (76).

8. The tool of any one of preceding claims, **characterised in that** it further comprises means (88) for displacing and positioning the mobile element (72) with respect to the plate (52), adapted to lead the bore (74) of the mobile element substantially in a chosen direction (C-C).

9. The tool of Claim 8, **characterised in that** the means (88) for displacing and positioning the mobile element (72) comprise a rigid support (90) for retaining the plate (52) and a rod (92) for driving the mobile element, adapted to be displaced with respect to the rigid support, said support being provided with two protractors (106, 112) adapted to plot the angular position of the rod (92) in two planes substantially perpendicular to each other.

## Patentansprüche

1. Hilfswerkzeug zum Einsetzen eines Gelenkpfannen-Implantats, von der Art, die eine Platte (52) aufweist, welche eine Auflagefläche (56) begrenzt, die dazu bestimmt ist, sich gegen die Gelenkpfanne eines Schulterblatts (2) eines Patienten anzulegen, und die eine Richtführung für ein Bohrorgan, ein Formgebungsorgan oder Ähnliches bildet, **dadurch gekennzeichnet, dass** es Einrichtungen (70) zum Einstellen der Richtung der Führung bezüglich der Platte (52) aufweist, die einerseits ein bezüglich der Platte bewegliches Element (72) und andererseits ein Element (76) zur Blockierung des beweglichen Elements (72) bezüglich der Platte aufweist, wobei das bewegliche Element mit einer geradlinigen Durchgangsbohrung (74) versehen ist, die geeignet ist, um die Richtführung für das Bohrorgan, das Formgebungsorgan oder Ähnliches zu bilden.

2. Hilfswerkzeug nach Anspruch 1, **dadurch gekennzeichnet, dass** die Einrichtungen (70) zur Einstellung der Richtung der Führung bestimmt sind, um in einer Durchgangsöffnung (60) aufgenommen und blockiert zu werden, die in der Platte (52) ausgebildet ist und auf der Auflagefläche (56) der Platte mündet.

3. Hilfswerkzeug nach einem der Ansprüche 1 oder 2, **dadurch gekennzeichnet, dass** die Einstelleinrichtungen (70) es ermöglichen, die Richtung der Führung bezüglich der Platte (52) mindestens in zwei Ebenen (X-X, Z-Z), (Y-Y, Z-Z) im Wesentlichen senkrecht zueinander einzustellen.

4. Hilfswerkzeug nach einem der vorhergehenden Ansprüche, **dadurch gekennzeichnet, dass** die Richtung (Z'Z') der Bohrung (74) des beweglichen Elements (72) stufenlos einstellbar ist.

5. Hilfswerkzeug nach einem der vorhergehenden Ansprüche, **dadurch gekennzeichnet, dass** das bewegliche Element (72) eine Fläche (72a) aufweist, die geeignet ist, um mit einer zugeordneten Fläche (64) der Platte (52) zusammenzuwirken.

6. Hilfswerkzeug nach Anspruch 5, **dadurch gekennzeichnet, dass** die Fläche (72a) des beweglichen Elements (72), die geeignet ist, um mit der zugeordneten Fläche (64) der Platte (52) zusammenzuwirken, im Wesentlichen kugelförmig ist.

7. Hilfswerkzeug nach einem der vorhergehenden Ansprüche, **dadurch gekennzeichnet, dass** es außerdem ein Werkzeug (86) zum Betätigen des Blockierelements (76) aufweist.

8. Hilfswerkzeug nach einem der vorhergehenden Ansprüche, **dadurch gekennzeichnet, dass** es außerdem Einrichtungen (88) zur Verschiebung und zur Positionierung des beweglichen Elements (72) bezüglich der Platte (52) aufweist, die geeignet sind, um die Bohrung (74) des beweglichen Elements im Wesentlichen gemäß einer ausgewählten Richtung (C-C) zu lenken.

9. Hilfswerkzeug nach Anspruch 8, **dadurch gekennzeichnet, dass** die Einrichtungen (88) zur Verschiebung und zur Positionierung des beweglichen Elements (72) einen starren Halteträger (90) für die Platte (52) und eine Stange (92) zum Antrieb des beweglichen Elements aufweisen, die bezüglich des starren Trägers verschiebbar ist, wobei der Träger mit zwei Winkelmessern (106, 112) versehen ist, die geeignet sind, um die Winkelstellung der Stange (92) in zwei im Wesentlichen zueinander senkrechten Ebenen zu übertragen.
